# EUROPÄISCHE PATENTSCHRIFT

(11) **EP 1 685 125 B1**
(45) Veröffentlichungstag und Bekanntmachung des Hinweises auf die Patenterteilung: **06.06.2012**
(21) Anmeldenummer: 04790859.5
(22) Anmeldetag: 26.10.2004
(51) Int. Cl.: C07D 401/12, C07D 207/32

(54) **Zur Behandlung proliferativer Krankheiten geeignete Pyrrolderivate**
Pyrrole derivatives useful for the treatment of proliferative diseases
Dérivés de pyrrole utiles pour le traitement de maladies proliferatives

(30) Priorität: 19.11.2003 DE 10354060
(43) Veröffentlichungstag der Anmeldung: 02.08.2006
(73) Patentinhaber: Merck Patent GmbH, 64293 Darmstadt (DE)
(72) Erfinder: FINSINGER, Dirk, 64291 Darmstadt (DE); BUCHSTALLER, Hans-Peter, 64347 Griesheim (DE); BURGDORF, Lars, 60389 Frankfurt am Main (DE); WIESNER, Matthias, 64342 Seeheim-Jugenheim (DE); AMENDT, Christiane, 64289 Darmstadt (DE); GRELL, Matthias, 64291 Darmstadt (DE); SIRRENBERG, Christian, 64289 Darmstadt (DE); ZENKE, Frank, 64291 Darmstadt (DE)
(86) Internationale Anmeldenummer: PCT/EP2004/012076
(87) Internationale Veröffentlichungsnummer: WO 2005/049603

(56) Entgegenhaltungen:
- WO-A-03/022833
- US-A1- 2004 138 269

## Beschreibung

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen, bei denen die Hemmung, Regulierung und/oder Modulation der Signaltransduktion von Kinasen, insbesondere der Serin/Threonin-Kinasen eine Rolle spielt, ferner pharmazeutische Zusammensetzungen, die diese Verbindungen enthalten, sowie die Verwendung der Verbindungen zur Behandlung kinasebedingter Krankheiten.

Die vorliegende Erfindung betrifft einzelne Verbindungen gemäß Anspruch 1 als Inhibitoren von Raf-Kinasen.

Protein-Phosphorylierung ist ein fundamentaler Prozess für die Regulation von Zellfunktionen. Die koordinierte Wirkung von sowohl Proteinkinasen als auch Phosphatasen kontrolliert die Phosphorylierungsgrade und folglich die Aktivität spezifischer Zielproteine. Eine der vorherrschenden Rollen der Protein-Phosphorylierung ist bei der Signaltransduktion, wenn extrazelluläre Signale amplifiziert und durch eine Kaskade von Protein-Phosphorylierungs- und Dephosphorylierungsereignissen, z. B. im p21^{ras}/raf-Weg propagiert werden.

Das p21^{ras}-Gen wurde als ein Onkogen der Harvey- und Kirsten-Ratten-Sarkom-Viren (H-Ras bzw. K-Ras) entdeckt. Beim Menschen wurden charakteristische Mutationen im zellulären Ras-Gen (c-Ras) mit vielen verschiedenen Krebstypen in Verbindung gebracht. Von diesen mutanten Allelen, die Ras konstitutiv aktiv machen, wurde gezeigt, dass sie Zellen, wie zum Beispiel die murine Zelllinie NIH 3T3, in Kultur transformieren.

Das p21^{ras}-Onkogen ist ein wichtiger beitragender Faktor bei der Entwicklung und Progression humaner solider Karzinome und ist bei 30 % aller humaner Karzinome mutiert (Bolton et al. (1994) Ann. Rep. Med. Chem., 29, 165-74; Bos. (1989) Cancer Res., 49, 4682-9). In seiner normalen, nicht mutierten Form ist das Ras-Protein ein Schlüsselelement der Signaltransduktionskaskade, die durch Wachstumsfaktor-Rezeptoren in fast allen Geweben gesteuert wird (Avruch et al. (1994) Trends Biochem. Sci., 19, 279-83).

Biochemisch ist Ras ein Guanin-Nukleotid-bindendes Protein, und das Zyklieren zwischen einer GTP-gebundenen aktivierten und einer GDPgebundenen ruhenden Form wird von Ras-endogener GTPase-Aktivität und anderen Regulatorproteinen strikt kontrolliert. Das Ras-Genprodukt bindet an Guanintriphosphat (GTP) und Guanindiphosphat (GDP) und hydrolysiert GTP zu GDP. Ras ist im GTP-gebundenen Zustand aktiv. In den Ras-Mutanten in Krebszellen ist die endogene GTPase-Aktivität abgeschwächt, und folglich gibt das Protein konstitutive Wachstumssignale an "Downstream"-Effektoren, wie zum Beispiel an das Enzym Raf-Kinase ab. Dies führt zum krebsartigen Wachstum der Zellen, die diese Mutanten tragen (Magnuson et al. (1994) Semin. Cancer Biol., 5, 247-53). Das Ras-Proto-Onkogen benötigt ein funktionell intaktes C-Raf-1 -Proto-Onkogen, um in höheren Eukaryoten durch Rezeptor- und Nicht-Rezeptor-Tyrosin-Kinasen initiierte Wachstums- und Differenzierungssignale zu transduzieren.

Aktiviertes Ras ist für die Aktivierung des C-Raf-1-Proto-Onkogens notwendig, die biochemischen Schritte, durch die Ras die Raf-1-Protein-(Ser/Thr)-Kinase aktiviert, sind jedoch inzwischen gut charakterisiert. Es wurde gezeigt, dass das Inhibieren des Effekts von aktivem Ras durch Inhibition des Raf-Kinase-Signalwegs mittels Verabreichung von deaktivierenden Antikörpern gegen Raf-Kinase oder mittels Koexpression dominanter negativer Raf-Kinase oder dominanter negativer MEK (MAPKK), dem Substrat der Raf-Kinase, zur Reversion transformierter Zellen zum normalen Wachstumsphänotyp führt, siehe: Daum et al. (1994) Trends Biochem. Sci., 19, 474-80; Fridman et al. (1994) J Biol. Chem., 269, 30105-8. Kolch et al. (1991) Nature, 349, 426-28) und zur Besprechung Weinstein-Oppenheimer et al. Pharm. & Therap. (2000), 88, 229-279.

Auf ähnliche Weise wurde die Inhibition von Raf-Kinase (durch Antisense-Oligodesoxynukleotide) in vitro und in vivo mit der Inhibition des Wachstums einer Reihe verschiedener humaner Tumortypen in Beziehung gebracht (Monia et al., Nat. Med. 1996, 2, 668-75; Geiger et al. (1997), Clin. Cancer Res. 3(7):1179-85; Lau et al. (2002), Antisense Nucl. Acid. Drug Dev. 12(1): 11-20; Mc Phillips et al. (2001), Br. J. Cancer 85(11): 1754-8).

Raf-Serin- und Threonin-spezifische Protein-Kinasen sind cytosolische Enzyme, die das Zellwachstum in einer Reihe verschiedener Zellsysteme stimulieren (Rapp, U.R., et al. (1988) in The Oncogene Handbook; T. Curran, E.P. Reddy und A. Skalka (Hrsg.) Elsevier Science Publishers; Niederlande, S. 213-253; Rapp, U.R., et al. (1988) Cold Spring Harbor Sym. Quant. Biol. 53:173-184; Rapp, U.R., et al. (1990) Inv Curr. Top. Microbiol. Immunol. Potter und Melchers (Hrsg.), Berlin, Springer-Verlag 166:129-139).

Drei Isozyme wurden charakterisiert:

C-Raf (Raf-1) (Bonner, T.I., et al. (1986) Nucleic Acids Res. 14:1009-1015). A-Raf (Beck, T.W., et al. (1987) Nucleic Acids Res. 15:595-609), und B-Raf (Qkawa, S., et al. (1998) Mol. Cell. Biol. 8:2651-2654; Sithanandam, G. et al. (1990) Oncogene:1775). Diese Enzyme unterscheiden sich durch ihre Expression in verschiedenen Geweben. Raf-1 wird in allen Organen und in allen Zelllinien, die untersucht wurden, exprimiert, und A- und B-Raf werden in Urogenital- bzw. Hirngeweben exprimiert (Storm, S.M. (1990) Oncogene 5:345-351).

Raf-Gene sind Proto-Onkogene: Sie können die maligne Transformation von Zellen initiieren, wenn sie in spezifisch veränderten Formen exprimiert werden. Genetische Veränderungen, die zu onkogener Aktivierung führen, erzeugen eine konstitutiv aktive Proteinkinase durch Entfernung oder Interferenz mit einer N-terminalen negativen Regulatordomäne des Proteins (Heidecker, G., et al. (1990) Mol. Cell. Biol. 10:2503-2512; Rapp, U.R., et al. (1987) in Oncogenes and Cancer; S. A. Aaronson, J. Bishop, T. Sugimura, M. Terada, K. Toyoshima und P. K. Vogt (Hrsg.) Japan Scientific Press, Tokyo). Mikroinjektion in NIH 3T3-Zellen von onkogen aktivierten, aber nicht Wildtyp-Versionen des mit Expressionsvektoren von Escherichia coli präparierten Raf-Proteins führt zu morphologischer Transformation und stimuliert die DNA-Synthese (Rapp, U.R., et al. (1987) in Oncogenes and Cancer; S. A. Aaronson, J. Bishop, T. Sugimura, M. Terada, K. Toyoshima, und P. K. Vogt (Hrsg.) Japan Scientific Press, Tokyo; Smith, M. R., et al. (1990) Mol. Cell. Biol. 10:3828-3833). Aktivierende Mutanten von B-Raf wurden in verschiedenen menschlichen Krebsarten identifiziert, z.B. des Darms, der Eierstöcke, Melanomen und Sarkomen (Davies, H. et al. (2002), Nature 417, 949-945; publiziert online 9. Juni 2002, 10.1038/nature00766). Überwiegende Mutation ist eine einzige phosphomimetische Substitution in der Kinase-Aktivierungsdomäne (V599E), die zu einer konstitutiven Kinaseaktivität und Transformation von NIH3T3-Zellen führt.

Der Vorgang der Angiogenese stellt die Entwicklung von neuen Blutgefäßen, in der Regel Kapillaren, aus dem bereits bestehenden Gefäßsystem dar. Angiogenese ist so definiert, dass sie (i) die Aktivierung von Endothelzellen; (ii) eine erhöhte Gefäßpermeabilität; (iii) anschließende Auflösung der Basalmembran und Extravasation von Plasmabestandteilen, die zur Bildung einer vorläufigen extrazellulären Fibringel-Matrix führen; (iv) die Proliferation und Mobilisierung von Endothelzellen; (v) die Reorganisation von mobilisierten Endothelzellen zur Bildung funktioneller Kapillaren; (vi) die Bildung des Kapillarschenkels und (vii) die Ablagerung einer Basalmembran sowie die Rekrutierung perivaskulärer Zellen zu neu gebildeten Gefäßen umfasst.

Eine normale Angiogenese wird während des Gewebswachstums von der Embryonalentwicklung bis zur Reifung aktiviert und tritt dann während des Erwachsenenlebens in einen Zeitraum relativer Ruhe ein.

Eine normale Angiogenese wird auch während der Wundheilung und in bestimmten Stadien des weiblichen Reproduktionszyklus' aktiviert. Unangemessene oder pathologische Angiogenese wurde mit verschiedenen Erkrankungszuständen in Verbindung gebracht, einschließlich verschiedener Retinopathien; ischämischer Erkrankung; Atherosklerose; chronischer entzündlicher Störungen; rheumatoider Arthritis und Krebs. Die Rolle der Angiogenese bei Erkrankungszuständen ist zum Beispiel in Fan et al., Trends in Pharmacol Sci. 16:54 66; Shawver et al., DOT Bd. 2, Nr. 2 Februar 1997; Folkmann, 1995, Nature Medicine 1:27-31 erläutert.

Bei Krebs wurde gezeigt, dass das Wachstum solider Tumore Angiogenese-abhängig ist. (Siehe Folkmann, J., J. Nat'l. Cancer Inst., 1990, 82, 4-6). Folglich ist die Ansteuerung pro-angiogenetischer Wege eine Strategie, die weitverbreitet verfolgt wird, um neue Therapeutika auf diesen Gebieten eines großen, unerfüllten medizinischen Bedarfs bereitzustellen.

Raf ist am Angiogenesevorgang beteiligt. Endotheliale Wachstumsfaktoren (z.B. Gefäßendothelwachstumsfaktor VEGF oder basischer Fibroblasten-Wachstumsfaktor bFGF) aktivierten Rezeptor-Tyrosinkinasen (z.B. VEGFR-2) und signalisieren durch die Ras/Raf/Mek/Erk-Kinasekaskade und schützen Endothelzellen vor Apoptose (Alavi et al. (2003), Science 301, 94-96; Hood, J.D., et al. (2002) Science 296, 2404; Mikula, M., et al. (2001) EMBO J. 20, 1952; Hauser, M., et al. (2001) EMBO J. 20, 1940; Wojnowski et al. (1997) Nature Genet. 16, 293). Die Aktivierung von VEGFR-2 durch VEGF ist ein entscheidender Schritt im Signalübertragungsweg, der die Tumorangiogenese einleitet. Die VEGF-Expression kann für Tumorzellen konstitutiv sein und kann auch als Antwort auf bestimmte Reize hochreguliert werden. Einer dieser Reize ist Hypoxie, wobei die VEGF-Expression sowohl in Tumor- als auch in benachbarten Wirtsgeweben hochreguliert wird. Der VEGF-Ligand aktiviert VEGFR-2 durch Bindung mit dessen extrazellulärer VEGF-Bindungsstelle. Dies führt zur Rezeptor-Dimerisierung von VEGFRs und zur Autophosphorylierung von Tyrosinresten an der intrazellulären Kinasedomäne von VEGFR-2. Die Kinasedomäne bewirkt einen Transfer eines Phosphats von ATP auf die Tyrosinreste, wodurch Bindungsstellen für Signalproteine stromabwärts von VEGFR-2 bereitgestellt werden, was schließlich zur Einleitung der Angiogenese führt (McMahon, G., The Oncologist, Bd. 5, Nr. 90001, 3-10, April 2000).

Mäuse mit einer gezielten Zerstörung im Braf-Gen sterben an Gefäßdefekten während der Entwicklung (Wojnowski, L., et al., 1997, Nature Genetics 16, Seite 293-296). Diese Mäuse zeigen Mängel in der Bildung des Gefäßsystems und in der Angiogenese, z.B. vergrößerte Blutgefäße und erhöhten apoptotischen Tod von differenzierten Endothelzellen.

Folglich ist aktiviertes Raf-1 ein intrazellulärer Aktivator des Zellwachstums. Raf-1-Protein-Serin-Kinase ist ein Kandidat für den "Downstream"-Effektor der Mitogen-Signaltransduktion, da Raf-Onkogene dem Wachstumsarrest begegnen, der aus einer Blockade zellulärer Ras-Aktivität aufgrund einer zellulären Mutation (Ras-revertante Zellen) oder Mikroinjektion von Anti-Ras-Antikörpern resultiert (Rapp, U.R., et al. (1988) in The Oncogene Handbook, T. Curran, E.P. Reddy und A. Skalka (Hrsg.), Elsevier Science Publishers; Niederlande, S. 213-253; Smith, M.R., et al. (1986) Nature (London) 320:540-543).

Die C-Raf-Funktion ist für die Transformation durch eine Reihe verschiedener Membran-gebundener Onkogene und für die Wachstumsstimulation durch in Sera enthaltene Mitogene erforderlich (Smith, M.R., et al. (1986) Nature (London) 320:540-543). Raf-1-Protein-Serin-Kinase-Aktivität wird durch Mitogene über die Phosphorylierung reguliert (Morrison, D.K., et al. (1989) Cell 58:648-657), welche auch die subzelluläre Verteilung bewirkt (Olah, Z., et al. (1991) Exp. Brain Res. 84:403; Rapp, U.R., et al. (1988) Cold Spring Harbor Sym. Quant. Biol. 53:173-184. Zu Raf-1-aktivierenden Wachstumsfaktoren zählen der aus Thrombozyten stammende Wachstumsfaktor (PDGF) (Morrison, D.K., et al. (1988) Proc. Natl. Acad. Sci. USA 85:8855-8859), der Kolonien-stimulierende Faktor (Baccarini, M., et al. (1990) EMBO J. 9:3649-3657), Insulin (Blackshear, P.J., et al. (1990) J. Biol. Chem. 265:12115-12118), der epidermale Wachstumsfaktor (EGF) (Morrison, R.K., et al. (1988) Proc. Natl. Acad. Sci. USA 85:8855-8859), Interleukin-2 (Turner, B.C., et al. (1991) Proc. Natl. Acad. Sci. USA 88:1227) und Interleukin-3 und der Granulozyten-Makrophagen-Kolonien-stimulierende Faktor (Carroll, M.P., et al. (1990) J. Biol. Chem. 265:19812-19817).

Nach der Mitogen-Behandlung von Zellen transloziert die transient aktivierte Raf-1-Protein-Serin-Kinase in den perinukleären Bereich und den Nukleus (Olah, Z., et al. (1991) Exp. Brain Res. 84:403; Rapp, U.R., et al. (1988) Cold Spring Habor Sym. Quant. Biol. 53:173-184). Zellen, die aktiviertes Raf enthalten, sind in ihrem Genexpressionsmuster verändert (Heidecker, G., et al. (1989) in Genes and signal transduction in multistage carcinogenesis, N. Colburn (Hrsg.), Marcel Dekker, Inc., New York, S. 339-374) und Raf-oncogenes activate transcription from Ap-I/PEA3-dependent promotors in transient transfection assays (Jamal, S., et al. (1990) Science 344:463-466; Kaibuchi, K., et al. (1989) J. Biol. Chem. 264:20855-20858; Wasylyk, C., et al. (1989) Mol. Cell. Biol. 9:2247-2250).

Es gibt mindestens zwei unabhängige Wege für die Raf-1-Aktivierung durch extrazelluläre Mitogene: Einen, der Proteinkinase C (KC) beinhaltet, und einen zweiten, der durch Protein-Tyrosin-Kinasen initiiert wird (Blackshear, P.J., et al. (1990) J. Biol. Chem. 265:12131-12134; Kovacina, K.S., et al. (1990) J. Biol. Chem. 265:12115-12118; Morrison, D.K., et al. (1988) Proc. Natl. Acad. Sci. USA 85:8855-8859; Siegel, J.N., et al. (1990) J. Biol. Chem. 265:18472-18480; Turner, B.C., et al. (1991) Proc. Natl. Acad. Sci. USA 88:1227). In jedem Fall beinhaltet die Aktivierung Raf-1-Protein-Phosphorylierung. Raf-1-Phosphorylierung kann eine Folge einer KinaseKaskade sein, die durch Autophosphorylierung amplifiziert wird, oder kann vollkommen durch Autophosphorylierung hervorgerufen werden, die durch Bindung eines vermutlichen Aktivierungsliganden an die Raf-1-Regulatordomäne, analog zur PKC-Aktivierung durch Diacylglycerol initiiert wird (Nishizuka, Y. (1986) Science 233:305-312).

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Protein-kinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

Die Synthese von kleinen Verbindungen, die die Signaltransduktion der Raf-Kinasen spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Insbesondere zeigen sie inhibierende Eigenschaften der Tyrosinkinase. Es wurde weiterhin gefunden, daß die erfindungsgemäßen Verbindungen Inhibitoren des Enzyms Raf-Kinase sind. Da das Enzym ein "Downstream"- Effektor von p21^{ras} ist, erweisen sich die Inhibitoren in pharmazeutischen Zusammensetzungen für die human- oder veterinärmedizinische Anwendung als nützlich, wenn Inhibition des Raf-Kinase-Weges, z. B. bei der Behandlung von Tumoren und/oder durch Raf-Kinase vermitteltem krebsartigen Zellwachstum, angezeigt ist. Die Verbindungen sind insbesondere nützlich bei der Behandlung solider Karzinome bei Mensch und Tier, z. B. von murinem Krebs, da die Progression dieser Krebse abhängig ist von der Ras-Protein-Signaltransduktionskaskade und deshalb auf die Behandlung durch Unterbrechung der Kaskade, d. h. durch Inhibition der Raf-Kinase, anspricht. Dementsprechend wird die erfindungsgemäßen Verbindung oder ein pharmazeutisch unbedenkliches Salz davon für die Behandlung von Krankheiten verabreicht, die durch den Raf-Kinase-Weg vermittelt werden, besonders Krebs, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom), pathologische Angiogenese und metastatische Zellmigration. Die Verbindungen sind ferner nützlich bei der Behandlung der Komplementaktivierungs-abhängigen chronischen Entzündung (Niculescu et al. (2002) Immunol. Res., 24:191-199) und durch HIV-1 (Human Immunodeficiency Virus Typ 1) induzierte Immunschwäche (Popik et al. (1998) J Virol, 72: 6406-6413), Infektionserkrankung, Influenza A virus (Pleschka, S. et al. (2001), Nat. Cell. Biol., 3(3):301-5) und Heliobacter pylori Infektion (Wessler, S. et al. (2002), FASEB J., 16(3): 417-9).

Es wurde überraschend gefunden, dass erfindungsgemäßen Verbindungen mit Signalwegen, besonders mit den hierin beschriebenen Signalwegen und bevorzugt dem Raf-Kinase-Signalweg interagieren können. Die erfindungsgemäßen Verbindungen zeigen bevorzugt eine vorteilhafte biologische Aktivität, die in auf Enzymen basierenden Assays, zum Beispiel Assays wie hierin beschrieben, leicht nachweisbar ist. In derartigen auf Enzymen basierenden Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Wie hierin besprochen, sind diese Signalwege für verschiedene Erkrankungen relevant. Dementsprechend sind die erfindungsgemäßen Verbindungen nützlich bei der Prophylaxe und/oder Behandlung von Erkrankungen, die von den genannten Signalwegen durch Interaktion mit einem oder mehreren der genannten Signalwege abhängig sind. Gegenstand der vorliegenden Erfindung sind deshalb erfindungsgemäße Verbindungen als Promotoren oder Inhibitoren, bevorzugt als Inhibitoren der hierin beschriebenen Signalwege. Bevorzugter Gegenstand der Erfindung sind deshalb erfindungsgemäße Verbindungen als Promotoren oder Inhibitoren, bevorzugt als Inhibitoren des Raf-Kinase-Weges. Ein bevorzugter Gegenstand der Erfindung sind deshalb erfindungsgemäße Verbindungen als Promotoren oder Inhibitoren, bevorzugt als Inhibitoren der Raf-Kinase. Ein noch bevorzugterer Gegenstand der Erfindung sind erfindungsgemäße Verbindungen als Promotoren oder Inhibitoren, bevorzugt als Inhibitoren einer oder mehrerer Raf-Kinasen, ausgewählt aus der Gruppe bestehend aus A-Raf, B-Raf und C-Raf-1. Ein besonders bevorzugter Gegenstand der Erfindung sind erfindungsgemäße Verbindungen als Promotoren oder Inhibitoren, bevorzugt als Inhibitoren von C-Raf-1.

Ein weiterer Gegenstand der vorliegenden Erfindung ist die Verwendung einer oder mehrerer erfindungsgemäßer Verbindungen zur Herstellung eines Arzneimittels zur Behandlung und/oder Prophylaxe von Erkrankungen, bevorzugt den hier beschriebenen Erkrankungen, die durch Raf-Kinasen veruracht, vermittelt und/oder propagiert werden und insbesondere Erkrankungen, die durch Raf-Kinasen ausgewählt aus der Gruppe, bestehend aus A-Raf, B-Raf and C-Raf-1 verursacht, vermittelt und/oder propagiert werden. Gewöhnlich werden die hier besprochenen Erkrankungen in zwei Gruppen eingeteilt, in hyperproliferative und nicht hyperproliferative Erkrankungen. In diesem Zusammenhang werden Psoriasis, Arthritis, Entzündungen, Endometriose, Vernarbung, gutartige Prostatahyperplasie, immunologische Krankheiten, Autoimmunkrankheiten und Immunschwächekrankheiten als nicht krebsartige Krankheiten angesehen, von denen Arthritis, Entzündung, immunologische Krankheiten, Autoimmunkrankheiten und Immunschwächekrankheiten gewöhnlich als nicht hyperproliferative Erkrankungen angesehen werden. In diesem Zusammenhang sind Hirnkrebs, Lungenkrebs, Plattenepithelkrebs, Blasenkrebs, Magenkrebs, zur herstellung eines Arzneimittels zur Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie als krebsartige Erkrankungen anzusehen, die alle gewöhnlich als hyperproliferative Erkrankungen angesehen werden. Insbesondere krebsartiges Zellwachstum und insbesondere durch Raf-Kinase vermitteltes krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.

Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-Gonzälez, R. und Glenney, Jr., J.R. 1992, J. Biol. Chem. 267, Seite 14535).

Zur Identifikation von Kinase-Inhibitoren stehen verschiedene AssaySysteme zur Verfügung z.B. Walters et al., Nature Drug Discovery 2003, 2; 259-266). Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J., 2002, 366,977-981).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

### STAND DER TECHNIK

### ZUSAMMENFASSUNG DER ERFINDUNG

Die Erfindung betrifft einzelne Verbindungen gemäß Anspruch 1 umfaßt von der Formel I 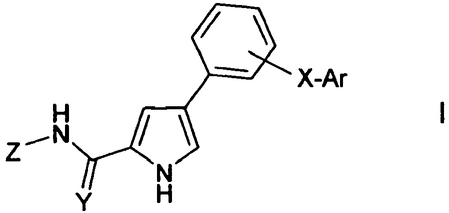 worin
- Ar: unsubstituiertes oder ein-, zwei-, drei-, vier- oder fünffach durch R¹ substituiertes Phenyl, Naphthyl, Biphenyl oder Het,
- X: -O-, -S-, -(CH₂)ₙ-, -C(=O)-, -CH(OH)-, -(CH₂)ₙO-, -O(CH₂)ₙ-, -(CH₂)ₙS-, -S(CH₂)ₙ-, -(CH₂)ₙNH-, -NH(CH₂)ₙ-, -(CH₂)ₙNA-, -NA(CH₂)ₙ-, -CHHal- oder -C(Hal)₂-,
- Y: O, S, CH-NO₂, C(CN)₂ oder N-R⁴,
- Z: -Ar, -Ar-X-Ar, -CH₂-Ar oder -CH₂-Ar-X-Ar,
- Het: ein- oder zweikerniger aromatischer Heterocyclus mit 1 bis 4 N-, O- und/oder S-Atomen,
- R¹: A, Ar', OR³, SR³, OAr', SAr', N(R³)₂, NHAr', Hal, NO₂. CN, (CH₂)ₘCOOR³, (CH₂)ₘCON(R³)₂, COR³, S(O)ₘA, S(O)ₘAr', NHCOA, NHCOAr', NHSO₂A, NHSO₂Ar', SO₂N(R³)₂, -O-(CH₂)ₚ-NH₂, -O-(CH₂)ₚ-NHA, -O-(CH₂)ₚ-NA₂, -NH-(CH₂)ₚ-NH₂, -NH-(CH₂)ₚ-NHA, -NH-(CH₂)ₚ-NA₂, -NA-(CH₂)ₚ-NH₂, -NA-(CH₂)ₚ-NHA, -NA-(CH₂)ₚ-NA₂, -O-(CH₂)ₙ-Het¹ oder Het¹,
- R³: H, A oder -(CH₂)ₙAr'.
- R⁴: H, CN, OH, A, (CH₂)ₘAr', COR³, COAr', S(O)ₘA oder S(O)ₘAr',
- Ar': unsubstituiertes oder ein-, zwei-, drei-, vier- oder fünffach durch A, Ph, OH, OA, SH, SA, OPh, SPh, NH₂, NHA, NA₂, NHPh, Hal, NO₂, CN, (CH₂)ₘCOOH, (CH₂)ₘCOOA, (CH₂)ₘCONH₂, (CH₂)ₘCONHA, CHO, COA, S(O)ₘA, S(O)ₘPh, NHCOA, NHCOPh, NHSO₂A, NHSO₂Ph oder SO₂NH₂ substituiertes Phenyl,
- Ph: unsubstituiertes oder ein-, zwei- oder dreifach durch A, Hal, CN, COOR, COOH, NH₂, NO₂, OH oder OA subsituiertes Phenyl,
- Het¹: einkerniger gesättigter Heterocyclus mit 1 bis 4 N-, O- und/oder S-Atomen, der unsubstituiert oder ein-, zwei- oder dreifach durch Hal, A, OA, CN, (CH₂)ₙOH, (CH₂)ₙHal, NH₂, =NH, =N-OH, =N-OA und/oder Carbonylsauerstoff (=O) substituiert sein kann,
- A: Alkyl mit 1 bis 10 C-Atomen, wobei auch 1-7 H-Atome durch F und/oder Chlor ersetzt sein können,
- Hal: F, Cl, Br oder I,
- n: 0, 1, 2 oder 3,
- m: 0, 1 oder 2,
- p: 1, 2, 3 oder 4
bedeuten,
sowie ihre pharmazeutisch verwendbaren Solvate, Salze und Stereoisomere, einschließlich deren Mischungen in allen Verhältnissen.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.

Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Die Verbindungen der Formel I gemäß Anspruch 1 und ihre Salze können durch ein Verfahren erhalten werden, dadurch gekennzeichnet, daß man
a) zur Herstellung von Verbindungen der Formel I, worin Y O bedeutet, eine Verbindung der Formel II 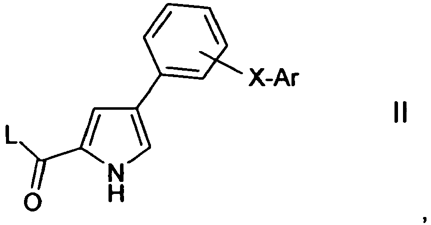 worin X und Ar die in Anspruch 1 angegebenen Bedeutungen haben, und L Cl, Br, I oder eine freie oder reaktionsfähig funktionell abgewandelte OH-Gruppe bedeutet,
   mit einer Verbindung der Formel III

   Z-NH₂ , III

   worin Z die in Anspruch 1 entsprechende Bedeutung hat,
   umsetzt,
   und/oder
   eine Base oder Säure der Formel I in eines ihrer Salze umwandelt.

Vor- und nachstehend haben die Reste X, Y, Z und Ar die bei der Formel **I** angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl. A bedeutet auch Cycloalkyl.

Cycloalkyl bedeutet vorzugsweise Cyclopropyl, Cyclobutyl, Cylopentyl, Cyclohexyl oder Cycloheptyl.

Alkylen ist vorzugsweise unverzweigt und bedeutet bevorzugt Methylen, Ethylen, Propylen, Butylen oder Pentylen.

R¹ bedeutet vorzugsweise z.B. A, wie z.B. Methyl oder Ethyl; Ar', wie z.B. Phenyl, F-, Cl- oder Bromphenyl oder Tolyl; OR³, wie z.B. Hydroxy, Methoxy oder Ethoxy; SR³, wie z.B. SCH₃; OAr', wie z.B. Phenoxy; SAr', wie z.B. S-Phenyl; N(R³)₂, wie z.B. Amino, Methylamino, Ethylamino, Dimethylamino oder Diethylamino; NHAr', wie z.B. Anilino; Hal, NO₂, CN, (CH₂)ₘCOOR³, wie z.B. Carboxy, Methoxycarbonyl, Methoxycarbonylmethyl oder Ethoxycarbonylethyl; (CH₂)ₘCON(R³)₂, wie z.B. Aminocarbonyl, N-Methylaminocarbonyl, Aminocarbonylmethyl oder Dimethylaminoethyl; COR³, wie z.B. Formyl, Acetyl oder Propionyl; S(O)ₘA, wie z.B. Methylsulfonyl; S(O)ₘAr', wie z.B. Phenylsulfonyl; NHCOA, wie z.B. Acetamino; NHCOAr', Phenylcarbonylamino; NHSO₂A, wie z.B. Methylsulfonylamino; NHSO₂Ar', wie z.B. Phenylsulfonylamino; SO₂N(R³)₂, wie z.B.Dimethylaminosulfonyl; -O-(CH₂)ₚ-NH₂, wie z.B. 2-Amino-ethoxy; -O-(CH₂)ₚ-NHA, wie z.B. 2-Methylamino-ethoxy; -O-(CH₂)ₚ-NA₂, wie z.B. 2-Dimethylamino-ethoxy; -NH-(CH₂)ₚ-NH₂, wie z.B. 2-Aminoethylamino; -NH-(CH₂)ₚ-NHA, wie z.B. 2-Methylamino-ethylamino; -NH-(CH₂)ₚ-NA₂, wie z.B. 2-Dimethylamino-ethylamino; -NA-(CH₂)ₚ-NH₂, wie z.B. (2-Amino-ethyl)-methyl-amino; -NA-(CH₂)ₚ-NHA, wie z.B. (2-Methylamino-ethyl)-methyl-amino; -NA-(CH₂)ₚ-NA₂, wie z.B. (2-Dimethylamino-ethyl)-methyl-amino; -O-(CH₂)ₙ-Het¹, wie z.B. 2-(Pyrrolidin-1-yl)-ethoxy, 2-(1-Piperidin-1-yl)-ethoxy, 2-(Morpholin-4-yl)-ethoxy, 2-(Piperazin-1-yl)-ethoxy, 2-(4-Methyl-piperazin-1-yl)-ethoxy, 2-(1-Methylpiperidin-4-yl)-ethoxy, 2-(4-Hydroxyethyl-piperazin-1-yl)-ethoxy oder 2-(4-Hydroxy-piperidin-1-yl)-ethoxy;
oder Het¹, wie z.B. 1-Pyrrolidinyl, 1-Piperidinyl, 4-Morpholinyl, 1-Piperazinyl, 4-Methyl-piperazin-1-yl, 4-Piperidinyl, 1-Methyl-piperidin-4-yl, 4-Hydroxyethyl-piperazin-1-yl, 4-Hydroxy-piperidin-1-yl.

Ar bedeutet vorzugsweise unsubstituiertes Phenyl, weiterhin ein-, zwei-, drei-, vier- oder fünffach durch A, Ph, OH, OA, SH, SA, OPh, SPh, NH₂, NHA, NA₂, NHPh, Hal, NO₂, CN, (CH₂)ₘCOOH, (CH₂)ₘCOOA, (CH₂)ₘCONH₂, (CH₂)ₘCONHA, CHO, COA, S(O)ₘA, S(O)ₘPh, NHCOA, NHCOPh, NHSO₂A, NHSO₂Ph oder SO₂NH₂ substituiertes Phenyl, wie z.B. o-, m- oder p-Tolyl, Biphenyl, o-, m- oder p-Hydroxyphenyl, o-, m- oder p-Methoxyphenyl, o-, m- oder p-Mercapto-phenyl, o-, m- oder p-Phenoxyphenyl, o-, m- oder p-Anilino, o-, m- oder p-Methylaminophenyl, o-, m-oder p-Phenylaminophenyl, o-, m- oder p-Fluorphenyl, o-, m- oder p-Chlorphenyl, o-, m- oder p-Bromphenyl, o-, m- oder p-Nitrophenyl, o-, m-oder p-Cyanphenyl, o-, m- oder p-Carboxyphenyl, o-, m- oder p-Carboxymethylphenyl, o-, m- oder p-Methoxycarbonylphenyl, o-, m- oder p-Methoxycarbonylmethylphenyl, o-, m- oder p-Aminocarbonylphenyl, o-, m-oder p-Methylaminocarbonylphenyl, o-, m- oder p-Formylphenyl, o-, m-oder p-Acetylphenyl, o-, m- oder p-Methylsulfonylphenyl, o-, m- oder p-Methylcarbonylaminophenyl, o-, m- oder p-Methylsulfonylaminophenyl, o-, m- oder p-Aminosulfonylphenyl, weiter bevorzugt 2,3-, 2,4-, 2,5-, 2,6-, 3,4-oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-Iodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl oder 3-Fluor-4-methoxyphenyl;
weiter, vorzugsweise, ungeachtet zusätzlicher Substitutionen z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4-oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7-oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzo-dioxol-5-yl, 1,4-Benzodioxan-6-yl, 2,1,3-Benzothiadiazol-4- oder -5-yl oder 2,1,3-Benzoxadiazol-5-yl.

Ar' bedeutet vorzugsweise z.B. unsubstituiertes Phenyl, weiterhin ein-, zwei-, drei-, vier- oder fünffach durch A, Ph, OH, OA, SH, SA, OPh, SPh, NH₂, NHA, NA₂, NHPh, Hal, NO₂, CN, (CH₂)ₘCOOH, (CH₂)ₘCOOA, (CH₂)ₘCONH₂, (CH₂)ₘCONHA, CHO, COA, S(O)ₘA, S(O)ₘPh, NHCOA, NHCOPh, NHSO₂A, NHSO₂Ph oder SO₂NH₂ substituiertes Phenyl, wie z.B. o-, m- oder p-Tolyl, Biphenyl, o-, m- oder p-Hydroxyphenyl, o-, m- oder p-Methoxyphenyl, o-, m- oder p-Mercapto-phenyl, o-, m- oder p-Phenoxyphenyl, o-, m- oder p-Anilino, o-, m- oder p-Methylaminophenyl, o-, m-oder p-Phenylaminophenyl, o-, m- oder p-Fluorphenyl, o-, m- oder p-Chlorphenyl, o-, m- oder p-Bromphenyl, o-, m- oder p-Nitrophenyl, o-, m-oder p-Cyanphenyl, o-, m- oder p-Carboxyphenyl, o-, m- oder p-Carboxymethylphenyl, o-, m- oder p-Methoxycarbonylphenyl, er, m- oder p-Methoxycarbonylmethylphenyl, o-, m- oder p-Aminocarbonylphenyl, o-, m-oder p-Methylaminocarbonylphenyl, o-, m- oder p-Formylphenyl, o-, m-oder p-Acetylphenyl, o-, m- oder p-Methylsulfonylphenyl, o-, m- oder p-Methylcarbonylaminophenyl, o-, m- oder p-Methylsulfonylaminophenyl, o-, m- oder p-Aminosulfonylphenyl, weiter bevorzugt 2,3-, 2,4-, 2,5-, 2,6-, 3,4-oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl oder 3-Fluor-4-methoxyphenyl.

Het bedeutet vorzugsweise z.B. 2- oder 3-Furyl, 2- oder 3-Thienyl, 1-, 2-oder 3-Pyrrolyl, 1-, 2, 4- oder 5-Imidazolyl, 1-, 3-, 4- oder 5-Pyrazolyl, 2-, 4-oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder - 5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6-oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyi, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothia-diazol-4- oder -5-yl oder 2,1,3-Benzoxadiazol-5-yl.

Als Substituent R¹ für Het ist Methylaminocarbonyl besonders bevorzugt.

In einer weiteren bevorzugten Ausführungsform bedeutet Het einen einkernigen gesättigten Heterocyclus mit 1 bis 3 N-, O- und/oder S-Atomen, besonders bevorzugt ist Pyridyl.

Unsubstituiertes Het¹ bedeutet vorzugsweise z.B. Tetrahydro-2- oder -3-furyl, 1,3-Dioxolan-4-yl, Tetrahydro-2- oder -3-thienyl, Tetrahydro-1-, -2-oder -4-imidazolyl, Pyrrolidinyl, Piperidinyl, Morpholinyl oder Piperazinyl.

Het¹ bedeutet besonders bevorzugt einen einkernigen gesättigten Heterocyclus mit 1 bis 2 N-Atomen, der unsubstituiert oder einfach durch A oder (CH₂)ₙOH substituiert sein kann.

Het¹ bedeutet ganz besonders bevorzugt 1-Pyrrolidinyl, 1-Piperidinyl, 4-Morpholinyl, 1-Piperazinyl, 4-Methyl-piperazin-1-yl, 4-Piperidinyl, 1-Methylpiperidin-4-yl, 4-Hydroxyethyl-piperazin-1-yl, 4-Hydroxy-piperidin-1-yl, 2-Oxo-piperidin-1-yl, 2-Oxo-pyrrolidin-1-yl, 5,5-Dimethyl-2-oxo-pyrrolidin-1-yl oder 3-Oxo-morpholin-4-yl.

Y bedeutet besonders bevorzugt O.

Z bedeutet vorzugsweise Ar, besonders bevorzugt unsubstituiertes oder ein-, zwei-, drei-, vier- oder fünffach durch A, OH, OA, NH₂, NHA, NA₂, -O-(CH₂)ₚ-NH₂, -O-(CH₂)ₚ-NHA, -O-(CH₂)ₚ-NA₂ -NH-(CH₂)ₚ-NH₂, -NH-(CH₂)ₚ-NHA, -NH-(CH₂)ₚ-NA₂, -NA-(CH₂)ₚ-NH₂, -NA-(CH₂)ₚ-NHA, -NA-(CH₂)ₚ-NA₂, -O-(CH2)ₙ-Het¹, Het¹ oder Hal substituiertes Phenyl.

In einer weiteren Ausführungsform bedeutet Z unsubstituiertes oder ein-, zwei-, drei-, vier- oder fünffach durch R¹ substituiertes Phenyl, -Phenylen-X-Ar, z.B. Phenylen-O-Het, -CH₂-Ar oder -CH₂-Phenylen-X-Ar, wobei Het z.B. unsubstituiertes oder einfach durch R¹ substituiertes Pyridyl bedeutet.

Hal bedeutet vorzugsweise F, Cl oder Br, aber auch I, besonders bevorzugt F oder Cl.

Für die gesamte Formel I gilt, daß sämtliche Reste, die mehrfach auftreten, wie z.B. R¹, gleich oder verschieden sein können, d.h. unabhängig voneinander sind.

Die Verbindungen der Formel I gemäß Anspruch 1 können ein oder mehrere chirale Zentren besitzen und daher in verschiedenen stereoisomeren Formen vorkommen. Die Formel I umschließt alle diese Formen.

Die Verbindungen der Formel I gemäß Anspruch 1 und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I gemäß Anspruch 1 umsetzt. Verbindungen der Formel I gemäß Anspruch 1 können vorzugsweise erhalten werden, indem man Verbindungen der Formel II mit Verbindungen der Formel III umsetzt.

Die Verbindungen der Formel II sind neu, die der Formel III sind in der Regel bekannt.

In den Verbindungen der Formel II bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.

Aktivierte Ester werden zweckmäßig in situ gebildet, z. B. durch Zusatz von HOBt oder N-Hydroxysuccinimid.

Vorzugsweise werden Verbindungen der Formel II eingesetzt, worin L OH bedeutet.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin oder Chinolin oder eines Überschusses der Carboxykomponente der Formel II.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa 0° und 150°, normaterweise zwischen 15° und 90°, besonders bevorzugt zwischen 15 und 30°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I gemäß Anspruch 1 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder ÖI-in-Wasser-Flüssigemulsionen oder Wasser-in-ÖI-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs-oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nicht-toxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I gemäß Anspruch 1 sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I gemäß Anspruch 1 sowie die Salze und Solvate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer ÖI-in-Wasser-Cremebasis oder einer Wasser-in-ÖI-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I gemäß Anspruch 1 hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per* se bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I gemäß Anspruch 1 und/oder ihre pharmazeutisch verwendbaren Salze, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I gemäß Anspruch 1 und/oder ihrer pharmazeutisch verwendbaren Salze, Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I gemäß Anspruch 1 und/oder ihrer pharmazeutisch verwendbaren Salze, Solvate und Stereoisomere, einschließlich deren Mischungen in allen Verhältnissen, und einer wirksamen Menge eines weiteren Arzneimittelwirkstoffs gelöst oder in lyophilisierter Form vorliegt.

### VERWENDUNG

Wie erläutert, sind die Signalwege für verschiedene Erkrankungen relevant. Folglich eignen sich die Pyrrol-Derivate, indem sie mit einem oder mehreren dieser Signalwege interagieren, zur Vorbeugung und/oder Behandlung von Erkrankungen, die von diesen Signalwegen abhängig sind.

Die erfindungsgemäßen Verbindungen sind bevorzugt Kinase-Modulatoren und bevorzugter Kinase-Inhibitoren. Erfindungsgemäß umfassen Kinasen, sind aber nicht beschränkt auf, eine oder mehrere Raf-Kinasen, eine oder mehrere Tie-Kinasen, eine oder mehrere VEGFR-Kinasen, eine oder mehrere PDGFR-Kinasen, p38-Kinase und/oder SAPK2alpha.

Gegenstand der Erfindung ist die Verwendung von Verbindungen der Formel I gemäß Anspruch 1, sowie ihrer pharmazeutisch verwendbaren Salze, Solvate und Stereoisomere, einschließlich deren Mischungen in allen Verhältnissen, zur Herstellung eines Arzneimittels zur Behandlung von Krankheiten, wobei die Erkrankungen ausgewählt sind aus der Gruppe der hyperproliferativen und nicht hyperproliferativen Erkrankungen.

Hierbei handelt es sich um Krebserkrankungen oder nicht krebsartige Erkrankungen.

Die nicht krebsartigen Erkrankungen sind ausgewählt aus der Gruppe bestehend aus Psoriasis, Arthritis, Entzündungen, Endometriose, Vernarbung, gutartiger Prostatahyperplasie, immunologischer Krankheiten, Autoimmunkrankheiten und Immunschwächekrankheiten.

Die krebsartigen Erkrankungen sind ausgewählt aus der Gruppe bestehend aus dermatologischen Tumoren, hämatologischen Tumoren, Sarkomen, Plattenepithelkrebs, Magenkrebs, Kopfkrebs, Halskrebs, Ösophaguskrebs, Lymphom, Eierstockkrebs, Gebärmutterkrebs und/oder Prostatakrebs. Eine Modulation des Raf-Kinase-Wegs spielt eine noch wichtigere Rolle bei verschiedenen Krebsarten, die eine konstitutive Aktivierung des Raf-Kinase-abhängigen Signalwegs zeigen, wie Melanom, Kolorektalkrebs, Lungenkrebs, Hirnkrebs, Pankreaskrebs, Brustkrebs, gynäkologischer Krebs, Eierstockkrebs, Schilddrüsenkrebs, chronische Leukämie und akute Leukämie, Blasenkrebs, Leberkrebs und/oder Nierenkrebs. Eine Modulation des Raf-Kinase-Wegs spielt auch eine wichtige Rolle bei Infektionskrankheiten, bevorzugt den vorstehend/nachstehend genannten Infektionskrankheiten und besonders bei Helicobacter-pylori-Infektionen während einer Magengeschwürerkrankung.

Einer oder mehrere der vorstehend/nachstehend genannten Signalwege und besonders der VEGFR-Kinanse-Weg spielt eine wichtige Rolle bei der Angiogenese. Folglich eignen sich aufgrund der Kinase-modulierenden oder -inhibierenden Eigenschaften der efindungsgemäßen Verbindungen die erfindungsgemäßen Verbindungen zur Prophylaxe und/oder Behandlung von pathologischen Vorgängen oder Erkrankungen, die durch Angiogenese verursacht, vermittelt und/oder gefördert werden, zum Beispiel durch Einleitung einer Anti-Angiogenese. Pathologische Vorgänge oder Erkrankungen, die durch Angiogenese verursacht, vermittelt und/oder gefördert werden, umfassen, sind aber nicht beschränkt auf Tumore, besonders solide Tumore, Arthritis, besonders rheumatische oder rheumatoide Arthritis, diabetische Retinopathie, Schuppenflechte, Restenose; fibrotische Störungen; Störungen der Mesangiumzellproliferation, diabetische Nephropathie, maligne Nephrosklerose, thrombotische Mikroangiopathie-Syndrome, Organtransplantatabstoßung, Glomerulopathien, Stoffwechselstörungen, Entzündung und neurodegenerative Erkrankungen und besonders solide Tumore, rheumatische Arthritis, diabetische Retinopathie und Schuppenflechte.

Eine Modulation des p38-Signalwegs spielt eine wichtige Rolle bei verschiedenen kanzerösen und auch bei verschiedenen nichtkanzerösen Störungen, wie Fibrose, Atherosklerose, Restenose, Gefäßerkrankung, Kardiovaskulärerkrankung, Entzündung, Nierenerkrankung und/oder Angiogenese, und besonders nichtkanzerösen Störungen, wie rheumatoider Arthritis, Entzündung, Autoimmunerkrankung, chronischer obstruktiver Lungenerkrankung, Asthma und/oder Reizdarm.

Eine Modulation des PDGF-Signalwegs spielt eine wichtige Rolle bei verschiedenen kanzerösen und auch bei verschiedenen nichtkanzerösen Störungen, wie rheumatoider Arthritis, Entzündung, Autoimmunerkrankung, chronischer obstruktiver Lungenerkrankung, Asthma und/oder Reizdarm, und besonders nichtkanzerösen Störungen, wie Fibrose, Atherosklerose, Restenose, Gefäßerkrankung, Kardiovaskulärerkrankung, Entzündung, Nierenerkrankung und/oder Angiogenese.

Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptor modulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1- piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.

"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.

"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.

"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.

Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll.

Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.

Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylen-dioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.

Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diaza-tetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

### ASSAYS

Die Assays sind aus der Literatur bekannt und können vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

Allgemein sind erfindungsgemäße Verbindungen als geeignete Kinase-Modulatoren und besonders als geeignete erfindungsgemäße Kinase-Inhibitoren anzusehen, wenn sie eine Wirkung oder eine Aktivität auf eine oder mehrere Kinasen, bevorzugt auf eine oder mehrere Raf-Kinasen, zeigen, die bevorzugt, bestimmt als IC₅₀-Wert, im Bereich von 100 µmol oder darunter, bevorzugt 10 µmol oder darunter, bevorzugter im Bereich von 3 µmol oder darunter, noch bevorzugter im Bereich von 1 µmol oder darunter und am stärksten bevorzugt im nanomolaren Bereich liegt. Besonders bevorzugt für die erfindungsgemäße Verwendung sind Kinase-Inhibitoren, wie vorstehend/nachstehend definiert, die eine Aktivität, bestimmt als IC₅₀-Wert, auf eine oder mehrere Raf-Kinasen, die bevorzugt A-Raf, B-Raf und C-Raf-1 umfassen oder aus A-Raf, B-Raf und C-Raf-1 bestehen und bevorzugter C-Raf-1 umfassen oder aus C-Raf-1 bestehen, im Bereich von 0,5 µmol oder darunter und besonderes im Bereich von 0,1 µmol oder darunter zeigen. In vielen Fällen ist ein IC₅₀-Wert am unteren Ende der angegebenen Bereiche vorteilhaft, und in einigen Fällen ist es sehr wünschenswert, dass der IC₅₀-Wert so klein wie möglich ist oder dass die IC₅₀-Werte so klein wie möglich sind, aber gewöhnlich sind IC₅₀-Werte, die zwischen den vorstehend angegebenen oberen Grenzen und einer unteren Grenze im Bereich von 0,0001 µmol, 0,001 µmol, 0,01 µmol oder sogar über 0,1 µmol liegen, ausreichend, damit sie die gewünschte pharmazeutische Aktivität zeigen. Die gemessenen Aktivitäten können jedoch je nach dem entsprechenden gewählten Testsystem oder Assay schwanken.

Alternativ kann die vorteilhafte biologische Aktivität der erfindungsgemäßen Verbindung in *in vitro* Assays, wie *in vitro* Proliferationsassays oder *in vitro* Wachstumsassays, demonstriert werden. Geeignete *in vitro* Assays sind im Stand der Technik bekannt, zum Beispiel aus der hier zitierten Literatur und den in der Literatur zitierten Bezugsstellen, oder können wie nachstehend beschrieben durchgeführt oder auf dazu analoge Weise entwickelt und/oder durchgeführt werden.

Als Beispiel für einen *in vitro* Wachstumsassay können menschliche Tumorzelllinien, zum Beispiel HCT116, DLD-1 oder MiaPaCa, die mutierte K-ras-Gene enthalten, in Standard-Proliferationsassays, zum Beispiel für das Verankerungs-anhängige Wachstum auf Kunststoff oder das Verankerungs-unabhängige Wachstum in Weichagar, verwendet werden. Menschliche Tumorzelllinien sind kommerziell erhältlich, zum Beispiel von ATCC (Rockville, MD), und können gemäß im Stand der Technik bekannten Verfahren, zum Beispiel in RPMI mit 10% hitzeinaktiviertem fötalem Rinderserum und 200 mM Glutamin, kultiviert werden. Zellkulturmedien, fötales Rinderserum und Hilfsstoffe sind kommerziell erhältlich, zum Beispiel von Invitrogen/Gibco/BRL (Karlsruhe, BRD) und/oder QRH Biosciences (Lenexa, KS). In einem Standard-Proliferationsassay für Verankerungs-anhängiges Wachstum kann man 3x10³ Zellen in Gewebekulturplatten mit 96 Vertiefungen überimpfen und sich anheften lassen, zum Beispiel über Nacht bei 37 °C in einem Brutschrank bei 5 % CO₂. Die Verbindungen können in Medien in Verdünnungsreihen titriert und zu den Zellkulturen in 96 Vertiefungen hinzugefügt werden. Man lässt die Zellen wachsen, zum Beispiel 1 bis 5 Tage, gewöhnlich mit einem Nachfüllen von frischen, die Verbindung enthaltenden Medien bei etwa der Hälfte der Dauer des Wachstumszeitraums, zum Beispiel an Tag 3, wenn man die Zellen für 5 Tage wachsen lässt. Die Proliferation kann durch im Stand der Technik bekannte Verfahren überwacht werden, wie durch Messung der Stoffwechselaktivität, zum Beispiel mit einem colorimetrischen Standard-XXT-Assay (Boehringer, Mannheim), der durch ein Standard-ELISA-Plattenlesegerät bei OD 490/560 gemessen wird, durch Messen des ³H-Thymidin-Einbaus in DNA nach einer 8stündigen Kultur mit 1µCi ³H-Thymidin, Ernten der Zellen auf Glasfasermatten unter Verwendung einer Zellerntevorrichtung und Messen des ³H-Thymidin-Einbau durch Flüssigszintillationszählung oder durch Färbetechniken, wie Kristallviolettfärbung. Andere geeignete zelluläre Assaysysteme sind im Stand der Technik bekannt.

Alternativ können für Verankerungs-unabhängiges Zellwachstum 1 x 10³ bis 3x10³ Zellen in 0,4 % Seaplaque-Agarose in RPMI-Vollmedien ausplattiert werden, wobei eine Bodenschicht, die nur 0,64 % Agar in RPMI-Vollmedien enthält, zum Beispiel in Gewebekulturschalen mit 24 Vertiefungen, überschichtet wird. Vollmedien plus Verdünnungsreihen von Verbindungen können zu den Vertiefungen gegeben und, zum Beispiel bei 37 °C in einem Brutschrank bei 5 % CO₂, für einen ausreichenden Zeitraum inkubiert werden, zum Beispiel 10-14 Tage, bevorzugt unter wiederholtem Nachfüllen von frischen, die Verbindung enthaltenden Medien, üblicherweise in Abständen von 3-4 Tagen. Koloniebildung und Gesamtzellmasse können überwacht werden, die durchschnittliche Koloniegröße und die Anzahl der Kolonien können gemäß im Stand der Technik bekannten Verfahren quantifiziert werden, zum Beispiel unter Verwendung von "Image Capture"-Technologie und Bildanalyse-Software. "Image Capture"-Technologie und Bildanalyse-Software, wie Image Pro Plus oder media Cybernetics.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

| | |
|---|---|
| Massenspektrometrie (MS): | El (Elektronenstoß-Ionisation) M⁺ |
| | FAB (Fast Atom Bombardment) (M+H)⁺ |
| | ESI (Electrospray lonization) (M+H)⁺ |

| | |
|---|---|
| APCI-MS (atmospheric pressure chemical ionization - mass spectrometry) (M+H)⁺. | |

### Beispiel 1

### Herstellung von 4-{4-[5-(4-Chlor-3-trifluormethyl-phenylcarbamoyl)-1H-pyrrol-3-yl]-phenoxy}-pyridin-2-carbonsäure-N-methylamid (6)

### 1.1 Synthese von [2-(4-Benzyloxyphenyl)-3-dimethylamino-allyliden]-dimethyl-ammoniumperchlorat (1)

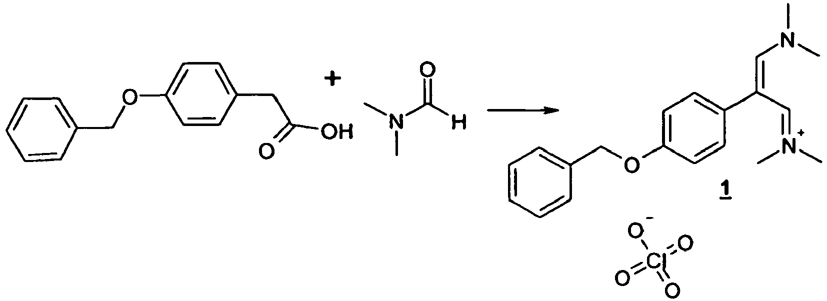

Zu einer Mischung von 6,7 ml Phosphorylchlorid (73,1 mmol) in 30 mL Dimethylformamid werden 6 g (4-Benzyloxy-phenyl)-essigsäure unter Schutzgasatmosphäre zugesetzt und 4 Stunden bei 70 °C gerührt. Nach Abkühlen wird das Lösungsmittel abgezogen, der Rückstand mit Eiswasser versetzt und 3,4 g Natriumperchlorat (24,4 mmol) gelöst in 20 mL Wasser zugegeben. Der ausgefallene Feststoff wird abfiltriert und mit Wasser gewaschen. Ausbeute: 9,9 g (98 %) 1, gelbe Kristalle.

### 1.2 Synthese von 4-(4-Benzyloxyphenyl)-1H-pyrrol-2-carbonsäureethylester (2)

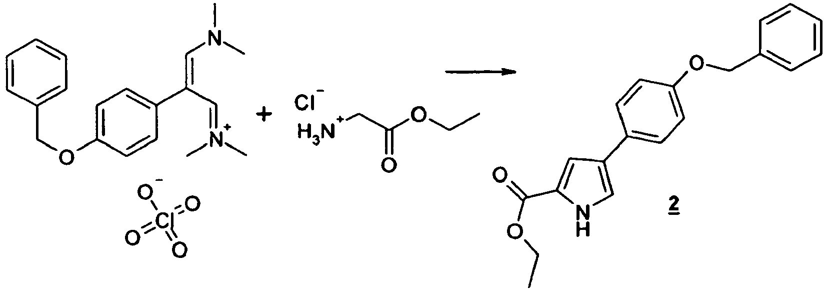

Zu einer Mischung von 10 mL einer 20%igen Natriumethylat-Lösung in Ethanol (27,4 mmol) und 2,3 g (16,4 mmol) Glycinethylesterhydrochlorid in 130 ml Ethanol werden 4,5 g [2-(4-Benzyloxy-phenyl)-3-dimethylamino-allyliden]-dimethyl-ammoniumperchlorat 1 unter Schutzgasatmosphäre zugesetzt und 24 Stunden unter Rückfluss erhitzt. Nach Abkühlen wird das Lösungsmittel abgezogen, der Rückstand in Wasser aufgenommen und das Produkt mit Essigsäureethylester extrahiert. Die vereinigten organischen Phasen werden über Natriumsulfat getrocknet. Das Produkt wird nach Abfiltrieren und Abdestillieren des Lösungsmittels erhalten. Ausbeute: 3,4 g (91 %) 2, braune Kristalle.

### 1.3 Synthese von 4-(4-Hydroxy-phenyl)-1 H-pyrrol-2-carbonsäure-ethylester (3)

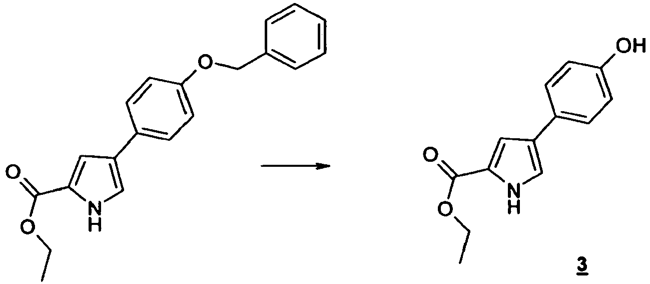

4,5 g (13,3 mmol) 4-(4-Benzyloxy-phenyl)-1*H*-pyrrol-2-carbonsäure-ethylester 2 werden in 90 mL Essigsäureethylester mit 1 g Katalysator (Palladium auf Aktivkohle 5%) versetzt und mit 0,3 I H₂ hydriert. Das Lösungsmittel wird abgezogen und das Produkt im Vakuum 1 Stunde bei 50C getrocknet.
Ausbeute: 2,8 g (91 %) 3, weisse Kristalle.

### 1.4 Synthese von 4-[4-(2-Methylcarbamoyl-pyridin-4-yloxy)-phenyl]-1 H-pyrrol-2-carbonsäureethylester (4)

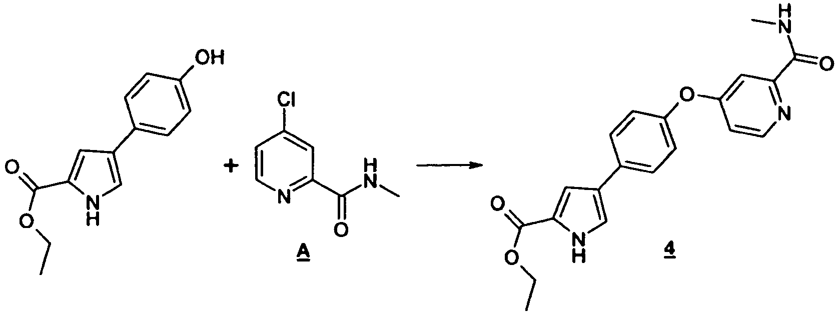

1,0 g (4,3 mmol) 4-(4-Hydroxy-phenyl)-1*H*-pyrrol-2-carbonsäureethylester 3 und 1,1g (6,5 mmol) 4-Chlor-pyridin-2-carbonsäure-N-methylamid A werden gut durchmischt und langsam auf 160°C erhitzt. Nach 48 Stunden bei 160 °C wird das Reaktionsgemisch nach Abkühlen bis knapp über den Ersrtarrungspunkt mit Essigsäureethylester versetzt und zweimal mit 2N Natronlauge und Wasser gewaschen. Nach Trocknung der organischen Phase und Abdestillieren des Lösungsmittels wird das Rohprodukt als braunes Öl erhalten. Dieses wird durch Normalphasensäulenchromatographie (Laufmittel Petrolether/Essigsäureethylester) gereinigt. Ausbeute: 0,7 g (40%) 4 gelbliche Kristalle.

### 1.5 Synthese von 4-[4-(2-Methylcarbamoyl-pyridin-4-yloxy)-phenyl]-1 H-pyrrol-2-carbonsäure (5)

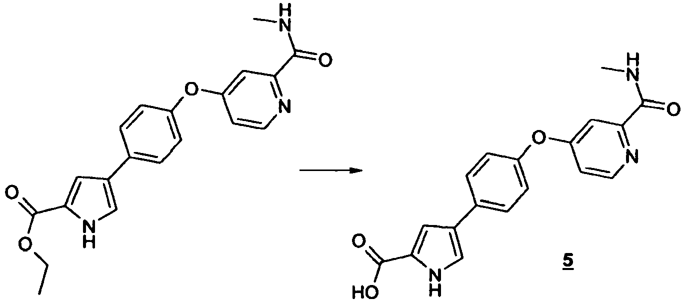

0,6 g (1,56 mmol) 4-[4-(2-Methylcarbamoyl-pyridin-4-yloxyl-phenyl]-1H-pyrrol-2-carbonsäureethylester 4 werden in 5 mL 2N Natronlauge und 20 mL Ethanol 16 Stunden bei 60°C gerührt. Nach Abdestillieren des Ethanols wird nach Neutralisieren mit konzentrierter Salzsäure mit Essigester extrahiert und die vereinigten organischen Phasen über Natriumsulfat getrocknet. Nach Abfiltrieren und Einengen wird das Produkt aus Methanol kristallisiert.
Ausbeute: 0,46 g (85%) 5 gelbe Kristalle.

### 1.6 Synthese von 4-{4-[5-(4-Chlor-3-trifluormethyl-phenylcarbamoyl)-1H-pyrrol-3-yl]-phenoxy}-pyridin-2-carbonsäure-N-methylamid (6)

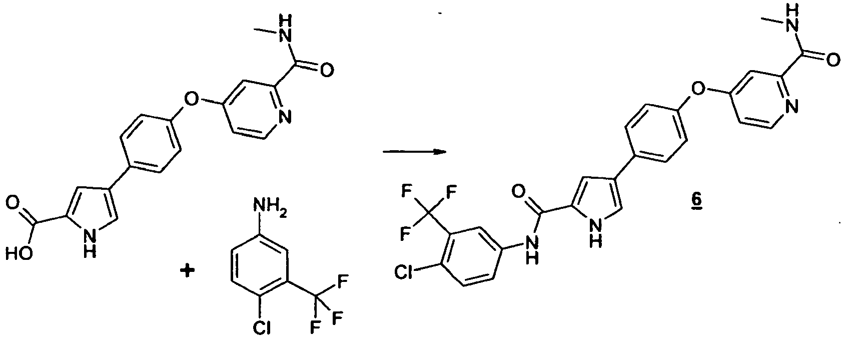

100 mg (0,3 mmol) 4-[4-(2-Methylcarbamoyl-pyridin-4-yloxy)-phenyl]-1 H-pyrrol-2-carbonsäure 5 werden in 3 mL Dimethylformamid gelöst und mit 61 mg (0,3 mmol) 5-Amino-2-chlorbenzotrifluorid, 57 mg (0,3 mmol) N(-3-Dimethylaminopropyl)-N'-ethylcarbodiimidhydrochlorid und 45,3 mg (0,3 mmol) 1-Hydroxybenzotriazolhydrat versetzt. Es wird 48 Stunden bei Raumtemperatur gerührt. Das Reaktionsgemisch wird mit Wasser versetzt und das Produkt mit Essigsäureethylester extrahiert. Die vereinigten organischen Phasen werden über Natriumsulfat getrocknet. Das Rohprodukt wird durch Normalphasensäulenchromatographie (Laufmittel Petrolether/Essigsäureethylester) gereinigt.
Ausbeute: 25 mg (17%) 6 gelbe Kristalle
HPLC-Retentionszeit tr [min]: 3.64
Bedingungen: Gradient 3.5 min
Flußrate: 1.5 ml/min von 80:20 nach 0:100 [H₂O/Acetonitril]
H₂O bzw. Acetonitril enthält 0.0 1 % TFA
Säule: Chromolith SpeedROD RP 18 e 50-4.6

Analog erhält man die nachstehenden Verbindungen
4-{3-[5-(4-Chlor-3-trifluormethyl-phenylcarbamoyl)-1*H*-pyrrol-3-yl]-phenoxy}-pyridin-2-carbonsäure-*N*-methylamid, tr 3.65; EI m/z 515;
4-{4-[5-(3-Chlor-4-methyl-phenylcarbamoyl)-1*H*-pyrrol-3-yl]-phenoxy}-pyridin-2-carbonsäure-*N*-methylamid, tr 3.48;
4-{4-[5-(2-Methoxy-5-trifluormethyl-phenylcarbamoyl)-1*H*-pyrrol-3-yl]-phenoxy}-pyridin-2-carbonsäure-*N*-methylamid, tr 3.54;
4-{3-[5-(3-Chlor-4-methyl-phenylcarbamoyl)-1*H*-pyrrol-3-yl]-phenoxy}-pyridin-2-carbonsäure-*N*-methylamid, tr 3.49; EI m/z 461;
4-{4-[5-(3-Chlor-6-methoxy-phenylcarbamoyl)-1*H*-pyrrol-3-yl]-phenoxy}-pyridin-2-carbonsäure-*N*-methylamid, tr 3.46;
4-{3-[5-(3-Chlor-6-methoxy-methyl-phenylcarbamoyl)-1*H*-pyrrol-3-yl]-phenoxy}-pyridin-2-carbonsäure-*N*-methylamid, tr 3.47; EI m/z 477;
4-{3-[5-(2-Methoxy-5-trifluormethyl-phenylcarbamoyl)-1*H*-pyrrol-3-yl]-phenoxy}-pyridin-2-carbonsäure-*N*-methylamid, tr 3.55; EI m/z 510;
4-{3-[5-(2,5-Dimethoxy-4-chlor-phenylcarbamoyl)-1*H*-pyrrol-3-yl]-phenoxy}-pyridin-2-carbonsäure-*N*-methylamid, tr 3.46; EI m/z 507;
4-{3-[5-(4-Brom-3-trifluormethyl-phenylcarbamoyl)-1*H*-pyrrol-3-yl]-phenoxy}-pyridin-2-carbonsäure-*N*-methylamid, tr 3.70; EI m/z 559;
4-{3-[5-(3-Trifluormethyl-phenylcarbamoyl)-1*H*-pyrrol-3-yl]-phenoxy}-pyridin-2-carbonsäure-*N*-methylamid, tr 3.50; EI m/z 480;
4-{3-[5-(4-Tert.-butyl-phenylcarbamoyl)-1*H*-pyrrol-3-yl]-phenoxy}-pyridin-2-carbonsäure-*N*-methylamid, tr 3.67; EI m/z 469;
4-{3-[5-(3,4-Dichlor-phenylcarbamoyl)-1*H*-pyrrol-3-yl]-phenoxy}-pyridin-2-carbonsäure-*N*-methylamid, tr 3.63; EI m/z 481;
4-{3-[5-(4-Chlor-3-methyl-6-methoxy-phenylcarbamoyl)-1*H*-pyrrol-3-yl]-phenoxy}-pyridin-2-carbonsäure-*N*-methylamid, tr 3.63; EI m/z 491;
4-{3-[5-(2,4-Dimethoxy-5-trifluormethoxy-phenylcarbamoyl)-1*H*-pyrrol-3-yl]-phenoxy}-pyridin-2-carbonsäure-*N*-methylamid, tr 3.69; EI m/z 540;
4-{3-[5-(2-Dimethylamino-5-trifluormethyl-phenylcarbamoyl)-1*H-*pyrrol-3-yl]-phenoxy}-pyridin-2-carbonsäure-*N*-methylamid, tr 3.77; EI m/z 524;
4-{3-[5-(2-(2-Methylamino-ethoxy)-5-methyl-phenylcarbamoyl)-1*H-*pyrrol-3-yl]-phenoxy}-pyridin-2-carbonsäure-*N*-methylamid, tr 2.65; EI m/z 500;
4-{3-[5-(2-(2-Dimethylamino-ethoxy)-5-methyl-phenylcarbamoyl)-1*H-*pyrrol-3-yl]-phenoxy}-pyridin-2-carbonsäure-*N*-methylamid, tr 2.45; EI m/z 514;
4-{3-[5-(2-[(2-Dimethylamino-ethyl)-methyl-amino]-5-methyl-phenylcarbamoyl)-1*H*-pyrrol-3-yl]-phenoxy}-pyridin-2-carbonsäure-*N-*methylamid, tr 2.34; EI m/z 527.

### Beispiel 2

Analog Beispiel 1 werden nachstehende Verbindungen erhalten
4-(3-Trifluormethyl-phenyl)-1*H*-pyrrol-2-carbonsäure-*N*-[4-(pyridin-4-yloxy)-phenyl]-amid 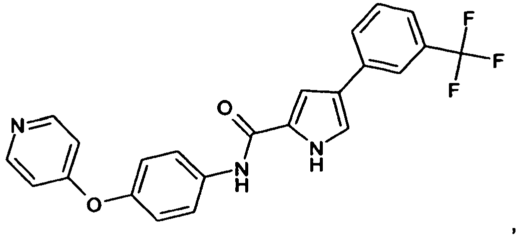
4-[4-({1-[4-(3-Trifluormethyl-phenyl)-1*H*-pyrrol-2-yl]-methanoyl}-amino)-phenoxy]-pyridin-2-carbonsäure-*N*-methylamid 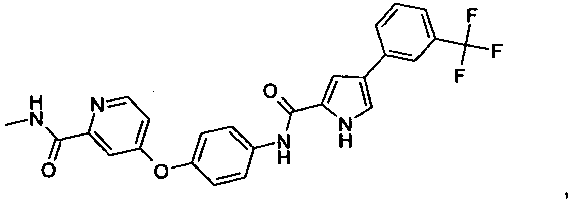
4-(3-Trifluormethyl-phenyl)-1*H*-pyrrol-2-carbonsäure-*N*-[4-(pyridin-3-yloxy)-phenyl]-amid,
4-(4-Phenoxy-phenyl)-1*H*-pyrrol-2-carbonsäure-*N*-[4-(pyridin-4-yloxy) phenyl]-amid,
4-[4-({1-[4-(4-Chlor-phenyl)-1*H*-pyrrol-2-yl]-methanoyl}-amino)-phenoxy]-pyridin-2-carbonsäure-*N*-methylamid,
4-(4-Chlor-phenyl)-1*H*-pyrrol-2-carbonsäure-*N*-[4-(pyridin-3-yloxy)-phenyl]-amid,
4-(4-Phenoxy-phenyl)-1*H*-pyrrol-2-carbonsäure-*N*-(3-trifluormethylphenyl)-amid,
4-(4-Phenoxy-phenyl)-1*H*-pyrrol-2-carbonsäure-*N*-(4-chlor-phenyl)-amid,
4-(4-Methylsulfonyl-phenyl)-1*H*-pyrrol-2-carbonsäure-*N*-[4-(pyridin-3-yloxy)-phenyl]-amid,
4-{4-[({1-[4-(4-Methylsulfonyl-phenyl)-1*H*-pyrrol-2-yl]-methanoyl}-amino)-methyl]-phenoxy}-pyridin-2-carbonsäure-*N*-methylamid,
4-(4-Methylsulfonyl-phenyl)-1*H*-pyrrol-2-carbonsäure-*N*-3-(pyridin-4-yloxy)-benzylamid.

Die nachfolgenden Beispiele betreffen Arzneimittel:

### Beispiel A: Injektionsgläser

Eine Lösung von 100 g eines Wirkstoffes der Formel I gemäß Anspruch 1 und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I gemäß Anspruch 1 mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I gemäß Anspruch 1, 9,38 g NaH₂PO₄ - 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I gemäß Anspruch 1 mit 99,5 g Vaseline unter aseptischen Bedingungen.

### Beispiel E: Tabletten

Ein Gemisch von 1 kg Wirkstoff der Formel I gemäß Anspruch 1, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I gemäß Anspruch 1 werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I gemäß Anspruch 1 in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
4-{4-[5-(4-Chlor-3-trifluormethyl-phenylcarbamoyl)-1*H*-pyrrol-3-yl]-phenoxy}-pyridin-2-carbonsäure-*N*-methylamid,
4-{3-[5-(4-Chlor-3-trifluormethyl-phenylcarbamoyl)-1*H*-pyrrol-3-yl]-phenoxy}-pyridin-2-carbonsäure-*N*-methylamid,
4-{4-[5-(3-Chlor-4-methyl-phenylcarbamoyl)-1*H*-pyrrol-3-yl]-phenoxy}-pyridin-2-carbonsäure-*N*-methylamid,
4-{4-[5-(2-Methoxy-5-trifluormethyl-phenylcarbamoyl)-1*H*-pyrrol-3-yl]-phenoxy}-pyridin-2-carbonsäure-*N*-methylamid,
4-{3-[5-(3-Chlor-4-methyl-phenylcarbamoyl)-1*H*-pyrrol-3-yl]-phenoxy}-pyridin-2-carbonsäure-*N*-methylamid,
4-{4-[5-(3-Chlor-6-methoxy-phenylcarbamoyl)-1*H*-pyrrol-3-yl]-phenoxy}-pyridin-2-carbonsäure-*N*-methylamid,
4-{3-[5-(3-Chlor-6-methoxy-phenylcarbamoyl)-1*H*-pyrrol-3-yl]-phenoxy}-pyridin-2-carbonsäure-*N*-methylamid,
4-{3-[5-(2-Methoxy-5-trifluormethyl-phenylcarbamoyl)-1*H*-pyrrol-3-yl]-phenoxy}-pyridin-2-carbonsäure-*N*-methylamid,
4-{3-[5-(2,5-Dimethoxy-4-chlor-phenylcarbamoyl)-1*H*-pyrrol-3-yl]-phenoxy}-pyridin-2-carbonsäure-*N*-methylamid,
4-{3-[5-(4-Brom-3-trifluormethyl-phenylcarbamoyl)-1*H*-pyrrol-3-yl]-phenoxy}-pyridin-2-carbonsäure-*N*-methylamid,
4-{3-[5-(3-Trifluormethyl-phenylcarbamoyl)-1*H*-pyrrol-3-yl]-phenoxy}-pyridin-2-carbonsäure-*N*-methylamid,
4-{3-[5-(4-Tert.-butyl-phenylcarbamoyl)-1*H*-pyrrol-3-yl]-phenoxy}-pyridin-2-carbonsäure-*N*-methylamid,
4-{3-[5-(3,4-Dichlor-phenylcarbamoyl)-1*H*-pyrrol-3-yl]-phenoxy}-pyridin-2-carbonsäure-*N*-methylamid,
4-{3-[5-(4-Chlor-3-methyl-6-methoxy-phenylcarbamoyl)-1*H-*pyrrol-3-yl]-phenoxy}-pyridin-2-carbonsäure-*N*-methylamid,
4-{3-[5-(2,4-Dimethoxy-5-trifluormethoxy-phenylcarbamoyl)-1*H-*pyrrol-3-yl]-phenoxy}-pyridin-2-carbonsäure-*N*-methylamid,
4-{3-[5-(2-Dimethylamino-5-trifluormethyl-phenylcarbamoyl)-1*H-*pyrrol-3-yl]-phenoxy}-pyridin-2-carbonsäure-*N*-methylamid,
4-{3-[5-(2-(2-Methylamino-ethoxy)-5-methyl-phenylcarbamoyl)-1 H-pyrrol-3-yl]-phenoxy}-pyridin-2-carbonsäure-*N*-methylamid,
4-{3-[5-(2-(2-Dimethylamino-ethoxy)-5-methyl-phenylcarbamoyl)-1*H*-pyrrol-3-yl]-phenoxy}-pyridin-2-carbonsäure-*N-*methylamid,
4-{3-[5-(2-[(2-Dimethylamino-ethyl)-methyl-amino]-5-methyl-phenylcarbamoyl)-1*H*-pyrrol-3-yl]-phenoxy}-pyridin-2-carbonsäure-*N-*methylamid,
sowie ihre pharmazeutisch verwendbaren Solvate, Salze und Stereoisomere, einschließlich deren Mischungen in allen Verhältnissen.

2. Arzneimittel, enthaltend mindestens eine Verbindung nach Anspruch 1 und/oder ihre pharmazeutisch verwendbaren Salze, Solvate und Stereoisomeren, einschließlich deren Mischungen in allen Verhältnissen, sowie gegebenenfalls Träger- und/oder Hilfsstoffe.

3. Verwendung von Verbindungen nach Anspruch 1 sowie ihrer pharmazeutisch verwendbaren Salze, Solvate und Stereoisomeren, einschließlich deren Mischungen in allen Verhältnissen, zur Herstellung eines Arzneimittels zur Behandlung von Krankheiten, wobei die Erkrankungen ausgewählt sind aus der Gruppe der hyperproliferativen und nicht hyperproliferativen Erkrankungen.

4. Verwendung nach Anspruch 3, wobei die Erkrankung Krebs ist.

5. Verwendung nach Anspruch 3, wobei die Erkrankung nicht krebsartig ist.

6. Verwendung nach Anspruch 5, wobei die nicht krebsartigen Erkrankungen ausgewählt sind aus der Gruppe bestehend aus Psoriasis, Arthritis, Entzündungen, Endometriose, Vernarbung, Heliobacter Pylori Infektion, Influenza A, gutartiger Prostatahyperplasie, immunologischer Krankheiten, Autoimmunkrankheiten und Immunschwächekrankheiten.

7. Verwendung nach Anspruch 3 oder 4, wobei die Erkrankungen ausgewählt sind aus der Gruppe bestehend aus Melanom, Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischem Krebs, Eierstockkrebs, Ovarkrebs, Gebärmutterkrebs, Prostatakrebs, Schilddrüsenkrebs, Lymphom, chronischer Leukämie und akuter Leukämie.

8. Verwendung nach Anspruch 3, wobei die Erkrankungen ausgewählt sind aus der Gruppe Arthritis, Restenose; fibrotischen Störungen; Störungen der Mesangiumzellproliferation, diabetischer Nephropathie, maligner Nephrosklerose, thrombotischen Mikroangiopathie-Syndromen, Organtransplantatabstoßung, Glomerulopathien, Stoffwechselstörungen, Entzündung, soliden Tumoren, rheumatischer Arthritis, diabetischer Neuropathie und neurodegenerativen Erkrankungen.

9. Verwendung nach Anspruch 3, wobei die Erkrankungen ausgewählt sind aus der Gruppe heumatoider Arthritis, Entzündung, Autoimmunerkrankung, chronischer obstruktiver Lungenerkrankung, Asthma, Reizdarm, Fibrose, Atherosklerose, Restenose, Gefäßerkrankung, Kardiovaskulärerkrankung, Entzündung, Nierenerkrankung und Angiogenesestörungen.

## Claims

1. Compounds selected from the group
*N*-methyl-4-{4-[5-(4-chloro-3-trifluoromethylphenylcarbamoyl)-1*H*-pyrrol-3-yl]phenoxy}pyridine-2-carboxamide,
*N*-methyl-4-{3-[5-(4-chloro-3-trifluoromethylphenylcarbamoyl)-1*H*-pyrrol-3-yl]phenoxy}pyridine-2-carboxamide,
*N*-methyl-4-{4-[5-(3-chloro-4-methylphenylcarbamoyl)-1*H*-pyrrol-3-yl]phenoxy}pyridine-2-carboxamide,
*N*-methyl-4-{4-[5-(2-methoxy-5-trifluoromethylphenylcarbamoyl)-1*H*-pyrrol-3-yl]phenoxy}pyridine-2-carboxamide,
*N*-methyl-4-{3-[5-(3-chloro-4-methylphenylcarbamoyl)-1*H*-pyrrol-3-yl]phenoxy}pyridine-2-carboxamide,
*N*-methyl-4-{4-[5-(3-chloro-6-methoxyphenylcarbamoyl)-1*H-*pyrrol-3-yl]phenoxy}pyridine-2-carboxamide,
*N*-methyl-4-{3-[5-(3-chloro-6-methoxymethylphenylcarbamoyl)-1*H*-pyrrol-3-yl]phenoxy}pyridine-2-carboxamide,
*N*-methyl-4-{3-[5-(2-methoxy-5-trifluoromethylphenylcarbamoyl)-1*H*-pyrrol-3-yl]phenoxy}pyridine-2-carboxamide,
*N*-methyl-4-{3-[5-(2,5-dimethoxy-4-chlorophenylcarbamoyl)-1*H-*pyrrol-3-yl]phenoxy}pyridine-2-carboxamide,
*N*-methyl-4-{3-[5-(4-bromo-3-trifluoromethylphenylcarbamoyl)-1*H*-pyrrol-3-yl]phenoxy}pyridine-2-carboxamide,
*N*-methyl-4-{3-[5-(3-trifluoromethylphenylcarbamoyl)-1*H*-pyrrol-3-yl]phenoxy}pyridine-2-carboxamide,
*N*-methyl-4-{3-[5-(4-tert-butylphenylcarbamoyl)-1*H*-pyrrol-3-yl]-phenoxy}pyridine-2-carboxamide,
*N*-methyl-4-{3-[5-(3,4-dichlorophenylcarbamoyl)-1*H*-pyrrol-3-yl]-phenoxy}pyridine-2-carboxamide,
*N*-methyl-4-{3-[5-(4-chloro-3-methyl-6-methoxyphenylcarba-moyl)-1*H*-pyrrol-3-yl]phenoxy}pyridine-2-carboxamide,
*N*-methyl-4-{3-[5-(2,4-dimethoxy-5-trifluoromethoxyphenylcarba-moyl)-1*H*-pyrrol-3-yl]phenoxy}pyridine-2-carboxamide,
*N*-methyl-4-{3-[5-(2-dimethylamino-5-trifluoromethylphenyl-carbamoyl)-1*H*-pyrrol-3-yl]phenoxy}pyridine-2-carboxamide,
*N*-methyl-4-{3-[5-(2-(2-methylaminoethoxy)-5-methylphenyl-carbamoyl)-1*H*-pyrrol-3-yl]phenoxy}pyridine-2-carboxamide,
*N*-methyl-4-{3-[5-(2-(2-dimethylaminoethoxy)-5-methylphenyl-carbamoyl)-1 H-pyrrol-3-yl]phenoxy}pyridine-2-carboxamide,
*N*-methyl-4-{3-[5-(2-[(2-dimethylaminoethyl)methylamino]-5-methylphenylcarbamoyl)-1*H*-pyrrol-3-yl]phenoxy}pyridine-2-carboxamide,
and pharmaceutically usable solvates, salts and stereoisomers thereof, including mixtures thereof in all ratios.

2. Medicaments comprising at least one compound according to Claim 1 and/or pharmaceutically usable salts, solvates and stereoisomers thereof, including mixtures thereof in all ratios, and optionally excipients and/or adjuvants.

3. Use of compounds according to Claim 1
and pharmaceutically usable salts, solvates and stereoisomers thereof, including mixtures thereof in all ratios, for the preparation of a medicament for the treatment of diseases, where the diseases are selected from the group of hyperproliferative and non-hyperproliferative diseases.

4. Use according to Claim 3, where the disease is cancer.

5. Use according to Claim 3, where the disease is non-cancerous.

6. Use according to Claim 5, where the non-cancerous diseases are selected from the group consisting of psoriasis, arthritis, inflammations, endometriosis, scarring, Heliobacter pylori infection, influenza A, benign prostate hyperplasia, immunological diseases, autoimmune diseases and immunodeficiency diseases.

7. Use according to one of Claims 3 or 4, where the diseases are selected from the group consisting of melanoma, brain cancer, lung cancer, squamous epithelium cancer, bladder cancer, stomach cancer, pancreatic cancer, liver cancer, kidney cancer, colorectal cancer, breast cancer, head cancer, neck cancer, oesophageal cancer, gynaecological cancer, ovarian cancer, cervical cancer, prostate cancer, thyroid cancer, lymphoma, chronic leukaemia and acute leukaemia.

8. Use according to Claim 3, where the diseases are selected from the group arthritis, restenosis; fibrotic disorders; disorders of mesangial cell proliferation, diabetic nephropathy, malignant nephrosclerosis, thrombotic microangiopathy syndromes, organ transplant rejection, glomerulopathies, metabolic disorders, inflammation, solid tumours, rheumatic arthritis, diabetic neuropathy and neurodegenerative diseases.

9. Use according to Claim 3, where the diseases are selected from the group rheumatoid arthritis, inflammation, autoimmune disease, chronic obstructive pulmonary disease, asthma, irritable bowel, fibrosis, atherosclerosis, restenosis, vascular disease, cardiovascular disease, inflammation, kidney disease and angiogenesis disorders.

## Revendications

1. Composés choisis dans le groupe constitué par
le *N*-méthyl-4-{4-[5-(4-chloro-3-trifluorométhylphénylcarbamoyl)-1*H*-pyrrol-3-yl]phénoxy}pyridine-2-carboxamide,
le N-méthyl-4-{3-[5-(4-chloro-3-trifluorométhylphénylcarbamoyl)-1*H*-pyrrol-3-yl]phénoxy}pyridine-2-carboxamide,
le *N*-méthyl-4-{4-[5-(3-chloro-4-méthylphénylcarbamoyl)-1*H-*pyrrol-3-yl]phénoxy}pyridine-2-carboxamide,
le *N*-méthyl-4-{4-[5-(2-méthoxy-5-trifluorométhylphénylcarba-moyl)-1*H*-pyrrol-3-yl]phénoxy}pyridine-2-carboxamide,
le *N*-méthyl-4-{3-[5-(3-chloro-4-méthylphénylcarbamoyl)-1*H-*pyrrol-3-yl]phénoxy}pyridine-2-carboxamide,
le *N*-méthyl-4-{4-[5-(3-chloro-6-méthoxyphénylcarbamoyl)-1*H-*pyrrol-3-yl]phénoxy}pyridine-2-carboxamide,
le *N*-méthyl-4-{3-[5-(3-chloro-6-méthoxyméthylphénylcarba-moyl)-1*H*-pyrrol-3-yl]phénoxy}pyridine-2-carboxamide,
le *N*-méthyl-4-{3-[5-(2-méthoxy-5-trifluorométhylphénylcarba-moyl)-1*H*-pyrrol-3-yl]phénoxy}pyridine-2-carboxamide,
le *N*-méthyl-4-{3-[5-(2,5-diméthoxy-4-chlorophénylcarbamoyl)-1*H*-pyrrol-3-yl]phénoxy}pyridine-2-carboxamide,
le *N*-méthyl-4-{3-[5-(4-bromo-3-trifluorométhylphénylcarbamoyl)-1*H*-pyrrol-3-yl]phénoxy}pyridine-2-carboxamide,
le *N*-méthyl-4-{3-[5-(3-trifluorométhylphénylcarbamoyl)-1*H-*pyrrol-3-yl]phénoxy}pyridine-2-carboxamide,
le *N*-méthyl-4-{3-[5-(4-tertio-butylphénylcarbamoyl)-1*H*-pyrrol-3-yl]phénoxy}pyridine-2-carboxamide,
le *N*-méthyl-4-{3-[5-(3,4-dichlorophénylcarbamoyl)-1*H*-pyrrol-3-yl]phénoxy}pyridine-2-carboxamide,
le *N*-méthyl-4-{3-[5-(4-chloro-3-méthyl-6-méthoxyphénylcarba-moyl)-1*H*-pyrrol-3-yl]phénoxy}pyridine-2-carboxamide,
le *N*-méthyl-4-{3-[5-(2,4-diméthoxy-5-trifluorométhoxyphényl-carbamoyl)-1 H-pyrrol-3-yl]phénoxy}pyridine-2-carboxamide,
le *N*-méthyl-4-{3-[5-(2-diméthylamino-5-trifluorométhylphényl-carbamoyl)-1 H-pyrrol-3-yl]phénoxy}pyridine-2-carboxamide,
le *N*-méthyl-4-{3-[5-(2-(2-méthylaminoéthoxy)-5-méthylphényl-carbamoyl)-1*H*-pyrrol-3-yl]phénoxy}pyridine-2-carboxamide,
le *N*-méthyl-4-{3-[5-(2-(2-diméthylaminoéthoxy)-5-méthylphényl-carbamoyl)-1*H*-pyrrol-3-yl]phénoxy}pyridine-2-carboxamide,
le *N*-méthyl-4-{3-[5-(2-[(2-diméthylaminoéthyl)méthylamino]-5-méthylphénylcarbamoyl)-1*H*-pyrrol-3-yl]phénoxy}pyridine-2-carbox-amide,
et les solvats, sels et stéréoisomères pharmaceutiquement utilisables de ceux-ci, y compris des mélanges de ceux-ci en toutes proportions.

2. Médicaments comprenant au moins un composé selon la revendication 1 et/ou des sels, solvats et stéréoisomères pharmaceutiquement utilisables de celui-ci, y compris des mélanges de ceux-ci en toutes proportions, et éventuellement des excipients et/ou des adjuvants.

3. Utilisation de composés selon la revendication 1 et de sels, solvats et stéréoisomères pharmaceutiquement utilisables de ceux-ci, y compris des mélanges de ceux-ci en toutes proportions, pour la préparation d'un médicament destiné au traitement de maladies, où les maladies sont choisies dans le groupe constitué par les maladies hyperprolifératives et non hyperprolifératives.

4. Utilisation selon la revendication 3, dans laquelle la maladie est le cancer.

5. Utilisation selon la revendication 3, dans laquelle la maladie est non cancéreuse.

6. Utilisation selon la revendication 5, dans laquelle les maladies non cancéreuses sont choisies dans le groupe constitué par le psoriasis, l'arthrite, les inflammations, l'endométriose, la cicatrisation, les infections par Heliobacter pylori, la grippe A, l'hyperplasie bénigne de la prostate, les maladies immunologiques, les maladies auto-immunes et les maladies d'immunodéficience.

7. Utilisation selon l'une des revendications 3 ou 4, dans laquelle les maladies sont choisies dans le groupe constitué par les mélanomes, le cancer du cerveau, le cancer du poumon, le cancer de l'épithélium pavimenteux simple, le cancer de la vessie, le cancer de l'estomac, le cancer du pancréas, le cancer du foie, le cancer du rein, le cancer colorectal, le cancer du sein, le cancer de la tête, le cancer du cou, le cancer de l'oesophage, le cancer gynécologique, le cancer de l'ovaire, le cancer du col de l'utérus, le cancer de la prostate, le cancer de la thyroïde, les lymphomes, la leucémie chronique et la leucémie aiguë.

8. Utilisation selon la revendication 3, dans laquelle les maladies sont choisies dans le groupe constitué par
l'arthrite, la resténose; les troubles fibrotiques; les troubles de prolifération des cellules mésangiales, la néphropathie diabétique, la néphrosclérose maligne, les syndromes de microangiopathie thrombotique, le rejet d'organes greffés, les glomérulopathies, les troubles métaboliques, les inflammations, les tumeurs solides, la polyarthrite rhumatoïde, la neuropathie diabétique et les maladies neurodégénératives.

9. Utilisation selon la revendication 3, dans laquelle les maladies sont choisies dans le groupe constitué par la polyarthrite rhumatoïde, les inflammations, les maladies auto-immunes, la broncho-pneumopathie chronique obstructive, l'asthme, le syndrome du côlon irritable, les fibroses, l'athérosclérose, la resténose, les maladies vasculaires, les maladies cardiovasculaires, les inflammations, les maladies rénales et les troubles d'angiogenèse.
